# EUROPEAN PATENT APPLICATION

(11) **EP 4 545 950 A1**
(43) Date of publication of application: **30.04.2025**
(21) Application number: 24199568.7
(22) Date of filing: 10.09.2024
(51) Int. Cl.: G01N 23/16, G01N 23/18, G01N 23/2206, G01N 23/223, G01N 33/00

(54) **X-RAY ANALYSIS APPARATUS**

(30) Priority: 25.10.2023 JP 2023183467
(71) Applicant: Hitachi High-Tech Science Corporation, Minato-ku Tokyo 105-6411 (JP)
(72) Inventor: GOTO, Ken, Tokyo, 105-6411, (JP); TAKAHARA, Toshiyuki, Tokyo, 105-6411, (JP)
(74) Representative: Zimmermann, Tankred Klaus

(57) **Abstract**

Proposed is an X-ray analysis apparatus capable of detecting a foreign matter and identifying elements in the foreign matter with high accuracy even when applied to a roll-to-roll method, without reducing throughput. The X-ray analysis apparatus includes an unwinding part (2) continuously unwinding a sample (S) downstream at a constant unwinding speed, a foreign matter inspection and analysis part (10) inspecting and analyzing a foreign matter in the sample unwound, and a winding part (7) winding the sample (S) that passed through the foreign matter inspection and analysis part (10). The foreign matter inspection and analysis part (10) includes a foreign matter location detection part (3) detecting a location of the foreign matter in the sample (S) that is unwound and moving, an intermittent transfer part (4) intermittently stopping or slowing movement of a portion of the sample (S) where the foreign matter is detected to a speed slower than the unwinding speed downstream of the foreign matter position detection part (3) when the foreign matter is detected, and an X-ray analysis part (5) irradiating, with X-rays, the portion where the foreign matter is detected that is stopped or moving at the speed than the unwinding speed and performing analysis. The intermittent transfer part (4) discharges the portion where the foreign matter is detected that is stopped or moving at the speed slower than the unwinding speed to the winding part (7) after the analysis.

## Description

### BACKGROUND OF THE INVENTION

### Technical Field

The present disclosure relates to an X-ray analysis apparatus capable of detecting the location of a foreign matter present in a sample in the form of a continuously transferred strip-shaped sheet, while performing high-accuracy analysis of the foreign matter.

### Description of the Related Art

Recently, demand for high-performance sheet materials, including battery materials, has been increasing. It is known that such high-performance sheet materials are wound into a roll shape and processed or inspected and manufactured using a roll-to-roll method.

As an example of the related art in this regard, Patent Document 1 discloses a transmission X-ray foreign matter inspection apparatus that inspects the presence of a foreign matter in a sample using transmission X-rays while continuously moving a roll-shaped or strip-shaped sample such as high-performance sheet material in a predetermined transfer direction.

In the transmission X-ray foreign matter inspection apparatus of Patent Document 1, while the foreign matter in the sample can be detected, the types of elements constituting the foreign matter cannot be identified.

As an example of the related art to overcome this problem, Patent Document 2 discloses an X-ray analysis apparatus that uses a combination of transmission X-rays and fluorescent X-rays. This X-ray analysis apparatus detects a foreign matter in a sheet-shaped sample using transmission X-rays, moves a sample stage to the location of the foreign matter based on the coordinate information of the foreign matter, and identifies elements in the foreign matter through fluorescent X-ray analysis.

### Documents of Related Art

(Patent Document 1) Japanese Patent Application Publication No. 2013-170924
(Patent Document 2) Japanese Patent No. 5956730

### SUMMARY OF THE INVENTION

However, the above-described related art technologies still have the following problems.

In the related art of Patent Document 2, a foreign matter was detected through use of transmission X-ray analysis, and then a sample was moving to a fluorescent X-ray inspection part and subjected to fluorescent X-ray analysis. When inspection was performed while moving the sample, the measurement time was shortened, making it difficult to measure the physical properties of the foreign matter with high accuracy. In particular, in the case of a sample in the form of a long strip-shaped sheet, it is necessary to cut a portion where the foreign matter is detected, move the portion to the fluorescent X-ray inspection part, and perform fluorescent X-ray analysis while stopping the transfer of the sample.

For this reason, there is a demand for an apparatus that performs detailed analysis and inspection, such as detection of a foreign matter in a sample transferred by a roll-to-roll method and identification of elements in the foreign matter, without reducing throughput.

Accordingly, the present disclosure has been made keeping in mind the above problems occurring in the related art, and an objective of the present disclosure is to provide an X-ray analysis apparatus capable of detecting a foreign matter and identifying elements in the foreign matter with high accuracy even when applied to a roll-to-roll method, without reducing throughput.

The present disclosure adopts the following configuration to solve the above problems. That is, an X-ray analysis apparatus according to the first disclosure may include: an unwinding part continuously unwinding a sample, which is a sheet in strip-shaped, downstream at a constant unwinding speed; a foreign matter inspection and analysis part inspecting and analyzing a foreign matter in the sample unwound from the unwinding part; and a winding part winding the sample that passed through the foreign matter inspection and analysis part. The foreign matter inspection and analysis part may include: a foreign matter location detection part detecting a location of the foreign matter in the sample unwound from the unwinding part and moving; an intermittent transfer part intermittently stopping or slowing movement of a portion of the sample where the foreign matter is detected to a speed slower than the unwinding speed downstream of the foreign matter location detection part when the foreign matter is detected by the foreign matter location detection part; and an X-ray analysis part irradiating, with X-rays, the portion where the foreign matter is detected that is stopped or moving at the speed slower than the unwinding speed and performing analysis. The intermittent transfer part may discharge the portion where the foreign matter is detected that is stopped or moving at the speed slower than the unwinding speed to the winding part while the portion after the analysis.

This X-ray analysis apparatus has the following characteristics. The foreign matter inspection and analysis part includes the foreign matter location detection part detecting the location of the foreign matter in the sample unwound from the unwinding part and moving, the intermittent transfer part intermittently stopping or slowing the movement of the portion of the sample where the foreign matter is detected to a speed slower than the unwinding speed downstream of the foreign matter location detection part when the foreign matter is detected by the foreign matter location detection part, and the X-ray analysis part irradiating, with X-rays, the portion where the foreign matter is detected that is stopped or moving at the speed slower than the unwinding speed and performing analysis. Therefore, the location of the foreign matter can be detected by the foreign matter location detection part while the sample is in a continuously moving state, and the foreign matter can be identified and analyzed for elements by the X-ray analysis part that performs fluorescent X-ray analysis while the portion where the foreign matter is detected is in a stopped or slowly moving state. Additionally, the intermittent transfer part discharges the portion where the foreign matter is detected that is stopped or moving at the speed slower than the unwinding speed to the winding part after analysis. Therefore, when analysis is completed, the portion in a stopped or slowly moving state is discharged and transferred toward a downstream side and wound on the winding part, thereby enabling X-ray analysis to be performed without entirely stopping the transfer of the sample.

As a result, even in a roll-to-roll method, it is possible to detect the location of the foreign matter while continuously moving the strip-shaped sample at a constant transfer speed, and to enable the portion where the foreign matter is detected to be stopped or moving slowly intermittently and partially. This enables analysis to be performed with high accuracy while securing sufficient measurement time without reducing the overall throughput.

According to an X-ray analysis apparatus according to the second disclosure, in the first disclosure, the foreign matter location detection part may be a transmission X-ray inspection part that detects the foreign matter by irradiating the sample with transmission X-rays, and the X-ray analysis part may be a fluorescent X-ray analysis part that detects fluorescent X-rays emitted from the sample by irradiating the portion where the foreign matter is detected with X-rays.

That is, this X-ray analysis apparatus has the following characteristics. The foreign matter location detection part is the transmission X-ray inspection part that detects the foreign matter by irradiating the sample with transmission X-rays, and the X-ray analysis part is the fluorescent X-ray analysis part that detects fluorescent X-rays emitted from the sample by irradiating the portion where the foreign matter is detected with X-rays. Therefore, the X-ray analysis part can perform elemental identification for the foreign matter with high accuracy by irradiating with X-rays the location of the foreign matter detected by the transmission X-rays and detecting the emitted fluorescent X-rays.

According to an X-ray analysis apparatus according to the third disclosure, in the first disclosure, the foreign matter location detection part may be a first transmission X-ray inspection part that detects the foreign matter by irradiating the sample with transmission X-rays, and the X-ray analysis part may be a second transmission X-ray inspection part that detects size of the foreign matter by irradiating the portion where the foreign matter is detected with X-rays.

That is, this X-ray analysis apparatus has the following characteristics. The foreign matter location detection part is the first transmission X-ray inspection part that detects the foreign matter by irradiating the sample with transmission X-rays, and the X-ray analysis part is the second transmission X-ray inspection part that detects the size of the foreign matter by irradiating the portion where the foreign matter is detected with transmission X-rays. Therefore, while the sample is moving, the first transmission X-ray inspection part (foreign matter location detection part) performs rough detection of only the location of the foreign matter, and then in a state where the sample is stopped or moving at a speed slower than the unwinding speed, the second transmission X-ray inspection part (X-ray analysis part) detects the size of the foreign matter. In this manner, a more detailed analysis of the foreign matter can be performed in two steps.

According to an X-ray analysis apparatus according to the fourth disclosure, in the first disclosure, the foreign matter location detection part may be a visible light inspection part that detects the foreign matter by detecting visible light from the sample, and the X-ray analysis part may be a fluorescent X-ray analysis part that detects fluorescent X-rays emitted from the sample by irradiating the portion where the foreign matter is detected with X-rays.

That is, this X-ray analysis apparatus has the following characteristics. The foreign matter location detection part is the visible light inspection part that detects the foreign matter by detecting visible light from the sample, and the X-ray analysis part is the fluorescent X-ray analysis part that detects fluorescent X-rays emitted from the sample by irradiating the portion where the foreign matter is detected with X-rays. Therefore, the visible light inspection part (foreign matter location detection part) can check the presence of a foreign matter, such as dust, wrinkles, bubbles, or discoloration, of the sample with visible light, and the fluorescent X-ray analysis part (X-ray analysis part) can identify elements constituting the foreign matter.

According to an X-ray analysis apparatus according to the fifth disclosure, in any one of the first to fourth disclosures, a marking part marking the portion where the foreign matter is detected or a vicinity thereof may be further provided.

That is, this X-ray analysis apparatus has the following characteristics. The marking part that marks the portion where the foreign matter is detected or the vicinity thereof is further provided. Therefore, the portion where the foreign matter is detected can be visually confirmed by marking, making it easier to analyze the foreign matter in subsequent processes.

According to an X-ray analysis apparatus according to the sixth disclosure, in the fifth disclosure, the marking part may perform marking in a state where the sample is stopped or moving at a speed slower than the unwinding speed by the intermittent transfer part.

That is, this X-ray analysis apparatus has the following characteristics. The marking part performs marking in a state where the sample is stopped or moving at a speed slower than the unwinding speed by the intermittent transfer part. Therefore, the portion where the foreign matter is detected can be marked directly and accurately in a stopped state or slowly moving state.

According to an X-ray analysis apparatus according to the seventh disclosure, in the fifth disclosure, the marking part may perform marking only on the portion where the foreign matter meeting predetermined conditions is detected or the vicinity thereof based on results of analysis of the foreign matter analyzed by the X-ray analysis part, or may perform marking with a specific mark.

That is, this X-ray analysis apparatus has the following characteristics. The marking part performs marking only on the portion where the foreign matter meeting predetermined conditions is detected or the vicinity thereof based on results of analysis of the foreign matter analyzed by the X-ray analysis part, or performs marking with a specific mark. Therefore, a portion where a foreign matter that do not cause a defective product is detected and a portion where a foreign matter that causes a defective product is detected can be discriminated by the presence of marking or the difference in marks.

For example, when the X-ray analysis part is the fluorescent X-ray analysis part, the portion where the foreign matter is detected may be marked only when a specific element that causes a defective product is detected from the constituent elements of the detected foreign matter, and when the X-ray analysis part is the transmission X-ray analysis part, the portion where the foreign matter is detected may be marked only when the detected foreign matter is of size that causes a defective product. As a result, even when foreign matters are detected, it is possible to visually distinguish between a foreign matter that does not cause a defective product and a foreign matter that causes a defective product, making it possible to improve the overall yield.

The present disclosure has the following effects.

That is, according to the X-ray analysis apparatus according to the present disclosure, the foreign matter inspection and analysis part includes the foreign matter location detection part detecting the location of the foreign matter in the sample unwound from the unwinding part and moving, the intermittent transfer part intermittently stopping or slowing the movement of the portion of the sample where the foreign matter is detected to a speed slower than the unwinding speed downstream of the foreign matter location detection part when the foreign matter is detected by the foreign matter location detection part, and the X-ray analysis part irradiating, with X-rays, the portion where the foreign matter is detected that is stopped or moving at a speed slower than the unwinding speed and performing analysis; and the intermittent transfer part discharges the portion where the foreign matter is detected that is stopped or moving at the speed slower than the unwinding speed to the winding part after analysis. Therefore, even in a roll-to-roll method, it is possible to detect the location of the foreign matter while continuously moving the strip-shaped sample at a constant transfer speed, and to enable the portion where the foreign matter is detected to be stopped or moving slowly intermittently and partially. This enables inspection of the physical properties of the foreign matter to be performed with high accuracy while securing sufficient measurement time without reducing the overall throughput.

As a result, the X-ray analysis apparatus according to the present disclosure can complete both identification of the location of a foreign matter and identification of elements in the foreign matter within a roll-to-roll process during manufacture of sheet materials.

### BRIEF DESCRIPTION OF THE DRAWINGS

The above and other objectives, features, and advantages of the present disclosure will be more clearly understood from the following detailed description taken in conjunction with the accompanying drawings, in which:
FIG. 1 is a configuration view illustrating an X-ray analysis apparatus according to a first embodiment according to the present disclosure;
FIGS. 2A, 2B, and 2C are explanatory views illustrating the state of an intermittent transfer part according to the first embodiment when detecting a foreign matter and stopping transfer (FIG. 2A), when starting transfer again from a stopped state (FIG. 2B), and when discharging a stored sample (FIG. 2C);
FIG. 3 is a configuration view illustrating an X-ray analysis apparatus according to a second embodiment according to the present disclosure; and
FIG. 4 is a configuration view illustrating an X-ray analysis apparatus according to a third embodiment according to the present disclosure.

### DESCRIPTION OF THE PREFERRED EMBODIMENTS

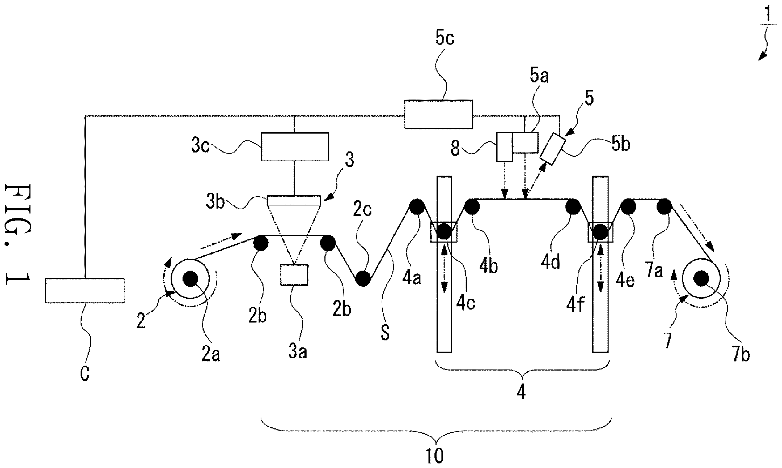

Hereinafter, a first embodiment of an X-ray analysis apparatus according to the present disclosure will be described with reference to FIGS. 1 to 2C.

As illustrated in FIGS. 1 to 2C, the X-ray analysis apparatus 1 according to this embodiment includes an unwinding part 2 continuously unwinding a sample S, which is a sheet in strip-shape, downstream at a constant unwinding speed; a foreign matter inspection and analysis part 10 inspecting and analyzing a foreign matter in the sample S unwound from the unwinding part 2; and a winding part 7 winding the sample S that passed through the foreign matter inspection and analysis part 10.

The foreign matter inspection and analysis part 10 includes a foreign matter location detection part 3 detecting the location of the foreign matter in the sample S unwound from the unwinding part 2 and moving; an intermittent transfer part 4 intermittently stopping or slowing the movement of a portion of the sample S where the foreign matter is detected to a speed slower than the unwinding speed downstream of the foreign matter location detection part 3 when the foreign matter is detected by the foreign matter location detection part 3; and an X-ray analysis part 5 irradiating, with X-rays, the portion where the foreign matter is detected that is stopped or moving at the speed slower than the unwinding speed and performing analysis.

The intermittent transfer part 4 is set to discharge the portion in a stopped or slowly moving state where the foreign matter is detected to the winding part after the analysis. That is, the intermittent transfer part 4 is set so that the portion in a stopped or slowly moving state where the foreign matter is detected is discharged to the winding part 7 at a higher speed than the unwinding speed in a short period of time after X-ray analysis and an unwinding amount of the sample S from the unwinding part 2 matches with a winding amount of the sample S onto the winding part 7.

Additionally, the X-ray analysis apparatus 1 according to this embodiment further includes a marking part 8 marking the portion where the foreign matter is detected or a vicinity thereof. The marking of the vicinity portion around the portion where the foreign matter is detected is achieved by, for example, marking a portion of the strip-shaped sample S where a side edge of the sample S meets an imaginary line extending in the width direction from the portion where the foreign matter is detected.

The marking part 8 performs marking in a state where the sample S is stopped or moving at a speed slower than the unwinding speed by the intermittent transfer part 4.

Additionally, the marking part 8 performs marking only on the portion where the foreign matter meeting predetermined conditions is detected or the vicinity thereof based on results of analysis of the foreign matter analyzed by the X-ray analysis part 5 or performs marking with a specific mark.

In this embodiment, the foreign matter location detection part 3 is a transmission X-ray inspection part (XRT inspection part) that detects the foreign matter by irradiating the sample S with transmission X-rays, and the X-ray analysis part 5 is a fluorescent X-ray analysis part (XRF analysis part) that detects fluorescent X-rays emitted from the sample S by irradiating the portion where the foreign matter is detected with X-rays.

The foreign matter location detection part 3 includes a first X-ray source 3a disposed below the transferred sample S and irradiating the sample S with X-rays; a first X-ray detector 3b disposed above the sample S to face the first X-ray source 3a and detecting X-rays passing through the sample S; and a foreign matter determination part 3c electrically connected to the first X-ray detector 3b, determining the presence and location of the foreign matter based on transmission X-rays detected by the first X-ray detector 3b and transmitting information on the location of the foreign matter.

The X-ray analysis part 5 includes a second X-ray source 5a disposed above the sample S of the intermittent transfer part 4 and irradiating the foreign matter in the sample S with X-rays; a second X-ray detector 5b disposed above the sample S of the intermittent transfer part 4 and detecting fluorescent X-rays emitted from the foreign matter; and a foreign matter analysis part 5c electrically connected to the second X-ray detector 5b, identifying elements in the foreign matter, and transmitting information on the identified element.

The unwinding part 2 includes an unwinding roller 2a for unwinding the sample S, on which the strip-shaped sample S before foreign matter detection is wound; a pair of foreign matter inspection rollers 2b horizontally arranged to horizontally transfer the sample S unwound from the unwinding roller 2a; and a first guide roller 2c guiding the sample S from the foreign matter inspection roller 2b to the intermittent transfer part 4.

While first X-ray source 3a is disposed below the sample S horizontally transferred between the pair of foreign matter inspection rollers 2b, the first X-ray detector 3b is disposed above the sample S.

The intermittent transfer part 4 includes first and second fixed rollers 4a and 4b guiding the sample S sent from the first guide roller 2c; a first moving roller 4c movable by a motor or the like in a direction orthogonal to a transfer path of the sample S (vertical direction in this embodiment) by passing through the space between the first and second fixed rollers 4a and 4b; third and fourth fixed rollers 4d and 4e guiding the sample S sent from the second fixed roller 4b; and a second moving roller 4f movable by a motor or the like in a direction orthogonal to the transfer path of the sample S (vertical direction in this embodiment) by passing through the space between the third and fourth fixed rollers 4d and 4e.

The second fixed roller 4b and the third fixed roller 4d are arranged horizontally to each other so that the sample S therebetween is transferred horizontally.

The first moving roller 4c is mounted on, for example, a ball screw (not illustrated) vertically extending to pass through the space between the first and second fixed rollers 4a and 4b, and is movable vertically by controlling a motor (not illustrated) that rotates the ball screw.

Additionally, similarly, the second moving roller 4f is mounted on, for example, a ball screw (not illustrated) vertically extending to pass through the space between the third and fourth fixed rollers 4d and 4e, and is movable vertically by controlling a motor (not illustrated) that rotates the ball screw.

The winding part 7 includes a second guide roller 7a guiding the sample S sent from the fourth fixed roller 4e, and a winding roller 7b winding the sample S from the second guide roller 7a at a constant winding speed. As described above, the transfer of the sample S in this embodiment is achieved by a roll-to-roll method. Additionally, the winding roller 7b is rotationally driven by a motor not illustrated.

The first X-ray detector 3b of the foreign matter location detection part 3 is, for example, a time delay integration (TDI) sensor that has multiple rows of line sensors along a specific direction and pixels arranged in a matrix form and serves as an X-ray detection part that detects X-rays passing through the sample S at the pixels.

The second X-ray detector 5b of the X-ray analysis part 5 is, for example, a silicon drift detector (SDD) in which a p-type semiconductor region is formed by injecting B (boron) into the window side of an n-type high-resistance substrate and a p-n junction is formed.

The X-ray analysis part 5 includes an analysis part moving mechanism not shown, such as an XY stage, moving the second X-ray source 5a and the second X-ray detector 5b along the X-Y axis (the X direction is the extension direction of the sample S, and the Y direction is the width direction of the sample S) with respect to the plane of the sample S.

Additionally, the X-ray analysis apparatus 1 according to this embodiment includes a controller C, which is a computer that controls the unwinding part 2, the intermittent transfer part 4, the foreign matter location detection part 3, the X-ray analysis part 5, the marking part 8, and the winding part 7 by electrically connecting thereto, and displays and records information on the location of the foreign matter received from the foreign matter location detection part 3 and information on the identified elements of the foreign matter received from the X-ray analysis part 5.

Next, foreign matter detection and foreign matter analysis methods for the sample S using the X-ray analysis apparatus 1 according to this embodiment will be described.

First, when the sample S is drawn out from the unwinding roller 2a of the unwinding part 2 at a constant unwinding speed and transferred to the location between the pair of foreign matter inspection rollers 2b, the sample S in a moving state is inspected for the presence of a foreign matter and the location of a foreign matter by the a foreign matter location detection part 3, which is a transmission X-ray inspection part.

When no foreign matter is detected in the sample S, the sample S is constantly unwound in a constant amount from the unwinding roller 2a at a constant unwinding speed, and the sample S between the second fixed roller 4b and the third fixed roller 4d is also transferred at the same unwinding speed. Additionally, since no foreign matter is detected, foreign matter analysis is not performed in the foreign matter analysis part 5c.

When a foreign matter is detected, the foreign matter location detection part 3 transmits the presence of the foreign matter in the sample S and the location of the foreign matter as foreign matter information to the controller C. Additionally, even when a plurality of impurities are detected, information on each foreign matter is transmitted to the controller C.

When a portion of the sample S where the foreign matter is detected is transferred to the location between the second fixed roller 4b and the third fixed roller 4d of the intermittent transfer part 4, the controller C controls the intermittent transfer part 4 to temporarily stop the transfer between the second fixed roller 4b and the third fixed roller 4d or to perform the transfer at a lower speed than the unwinding speed based on the received foreign matter information.

For example, when stopping the transfer of the sample S, a measurement time for fluorescent X-ray analysis is set to, for example, 2 seconds, for one detected foreign matter, and the transfer of the sample S is stopped by the intermittent transfer part 4 for a period of time according to the number of impurities.

When the transfer of the sample S is stopped by the intermittent transfer part 4, the rotation of the third fixed roller 4d is stopped first, and then the transfer of the sample S between the second fixed roller 4b and the third fixed roller 4d is stopped so that the sample S becomes a stopped state. At this time, the rotation of the unwinding roller 2a, which is driven to rotate at a constant rotation speed, is not stopped. When the next transfer is resumed, the sample S is constantly unwound in a constant amount from the unwinding roller 2a, and the sample S constantly unwound in the constant amount is stored by moving the first moving roller 4c.

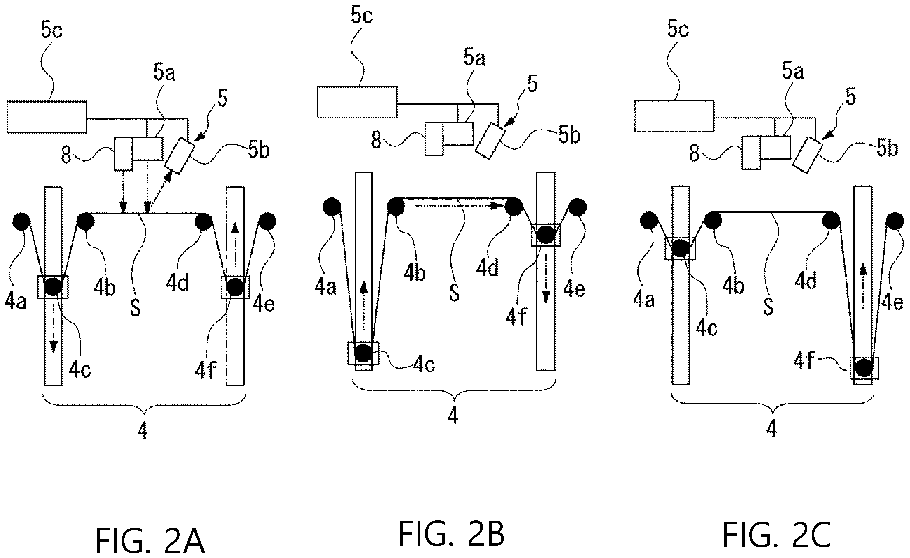

That is, as illustrated in FIG. 2A, as the first moving roller 4c on which the sample S is wound between the first fixed roller 4a and the second fixed roller 4b is slowly moving away from the first and second fixed rollers 4a and 4b in a direction orthogonal to the transfer path of the sample S (downward in this embodiment), the sample S is stored for the resumption of the next transfer. At this time, the sample S is stored between the first fixed roller 4a and the second fixed roller 4b by a length twice the distance between the first and second fixed rollers 4a and 4b and the first moving roller 4c. Additionally, the length of the temporarily stored sample S corresponds to a time period during which transfer is stopped for foreign matter analysis, and is determined so that the overall throughput of the sample S is not reduced.

Additionally, simultaneously with the above operation, the second moving roller 4f is gradually brought closer to the third and fourth fixed rollers 4d and 4e.

In a state where the transfer of the sample S is stopped by the intermittent transfer part 4 or is at a lower speed than the unwinding speed, the X-ray analysis part 5 performs fluorescent X-ray analysis on the foreign matter based on the foreign matter information. At this time, the second X-ray source 5a and the second X-ray analyzer 5b are moved by the analysis part moving mechanism, such as an XY stage, to the location of the foreign matter in the sample S that is in a stopped or slowly moving state and then irradiate the foreign matter with X-rays in a pin-point manner, and the foreign matter analysis part 5c identifies the elements of the foreign matter from fluorescent X-rays emitted and detected from the foreign matter.

Meanwhile, the marking part 8 marks a location where the foreign matter is detected or a vicinity location thereof.

For example, in a case where a Cu element contained in the foreign matter does not cause a defective product, but an Fe element contained in the foreign matter causes a defective product, when Cu is detected from the foreign matter by the X-ray analysis part 5, the marking part 8 does not perform marking or makes a mark indicating OK (positive) (e.g., ^{┌}∘_{┘} ), and when Fe is detected from the foreign matter by the X-ray analysis part 5, the marking part 8 performs marking or makes a mark indicating NG (defective) (e.g., ^{┌}×_{┘} ). Additionally, the marking part 8 is also movable to mark the location where the foreign matter is detected or the vicinity location thereof by the analysis part moving mechanism such as an XY stage.

Next, after the foreign matter analysis by the foreign matter analysis part 5c is completed, the first moving roller 4c is rapidly brought close to the first and second fixed rollers 4a and 4b in a short period of time in order to discharge a portion of the sample S stored by the first moving roller 4c in a short period of time.

That is, by rotating the third fixed roller 4d, the first moving roller 4c is brought close to the first and second fixed rollers 4a and 4b by a distance corresponding to half the length of the sample S to be transferred. The approach of the first moving roller 4c is performed in synchronization with the rotation of the third fixed roller 4d.

The sample S is wound around the first moving roller 4c in a U shape. The sample S is unwound from the second fixed roller 4b by a length corresponding to the sum of a length twice a moving distance of the first moving roller 4c and a length at which the sample S is unwound from the unwinding roller 2a in a short period of time.

Additionally, the sample S unwound from the second fixed roller 4b and transferred via the third fixed roller 4d is stored in a short period of time by the movement of the second moving roller 4f.

That is, as illustrated in FIG. 2B, as the second moving roller 4f on which the sample S is wound between the third fixed roller 4d and the fourth fixed roller 4e is rapidly moved away from the third and fourth fixed rollers 4d and 4e in a short period of time in a direction orthogonal to the transfer path of the sample S (downward in this embodiment), the sample S is stored for the discharge and winding.

The third fixed roller 4d is rotationally driven by a motor to collect a portion of the sample S for which foreign matter analysis has been completed, and the sample S is stored between the third and fourth fixed rollers 4d and 4e by a length twice the distance between the third and fourth fixed rollers 4d and 4e and the second moving roller 4f.

At this time, the rotation of the third fixed roller 4d and the movement of the second moving roller 4f are performed in synchronization with each other.

The sample S is wound around the second moving roller 4f in a U shape. The sample S is stored between the third fixed roller 4d and the fourth fixed roller 4e by a length corresponding to the sum of a length twice a moving distance of the second moving roller 4f and a length at which the sample S is wound on the winding roller 7b in a short period of time.

Additionally, the moving distance of the first moving roller 4c and the moving distance of the second moving roller 4f become the same.

FIG. 2C illustrates a state immediately after the sample S in a stopped location between the second fixed roller 4b and the third fixed roller 4d is transferred and stored between the third fixed roller 4d and the fourth fixed roller 4e.

Thereafter, the stored sample S is discharged by moving the second moving roller 4f and wound on the winding part 7. That is, as the second moving roller 4f between the third fixed roller 4d and the fourth fixed roller 4e is moved upward to be brought close to the third and fourth fixed rollers 4d and 4e, the stored sample S is discharged downstream from the fourth fixed roller 4e and wound on the winding roller 7b that is rotationally driven by a motor. Additionally, the winding roller 7b is set to constantly wind the sample S in a constant amount.

As described above, the X-ray analysis apparatus 1 according to this embodiment has the following characteristics. The foreign matter inspection and analysis part 10 includes the foreign matter location detection part 3 detecting the location of the foreign matter in the sample S unwound from the unwinding part 2 and moving, the intermittent transfer part 4 intermittently stopping or slowing the movement of the portion of the sample S where the foreign matter is detected to a speed slower than the unwinding speed downstream of the foreign matter location detection part 3 when the foreign matter is detected by the foreign matter location detection part 3, and the X-ray analysis part 5 analyzing the portion where the foreign matter is detected in a state where the potion is stopped or moving at a speed slower than the unwinding speed by irradiating the portion with X-rays. Therefore, the location of the foreign matter can be detected by the foreign matter location detection part 3 while the sample S is in a continuously moving state, and the foreign matter can be identified and analyzed for elements by the X-ray analysis part 5 that performs fluorescent X-ray analysis while the portion where the foreign matter is detected is in a stopped or slowly moving state.

In particular, the foreign matter location detection part 3 is a transmission X-ray inspection part that detects the foreign matter by irradiating the sample S with transmission X-rays, and the X-ray analysis part 5 is a fluorescent X-ray analysis part that detects fluorescent X-rays emitted from the sample S by irradiating the portion where the foreign matter is detected with X-rays. Therefore, the X-ray analysis part 5 can perform elemental identification for the foreign matter with high accuracy by irradiating with X-rays the location of the foreign matter detected by the transmission X-rays and detecting the emitted fluorescent X-rays.

Additionally, the intermittent transfer part 4 discharges the portion where the foreign matter is detected that is stopped or moving at the speed slower than the unwinding speed to the winding part 7 after analysis. Therefore, when analysis is completed, the portion in a stopped or slowly moving state is discharged and transferred toward a downstream side and wound on the winding part 7, thereby enabling X-ray analysis to be performed without entirely stopping the transfer of the sample S.

As a result, even in a roll-to-roll method, it is possible to detect the location of the foreign matter while continuously moving the strip-shaped sample S at a constant transfer speed, and to enable the portion where the foreign matter is detected to be stopped or moving slowly intermittently and partially. This enables analysis to be performed with high accuracy while securing sufficient measurement time without reducing the overall throughput.

Additionally, the marking part 8 that marks the portion where the foreign matter is detected or the vicinity thereof is further provided. Therefore, the portion where the foreign matter is detected can be visually confirmed by marking, making it easier to analyze the foreign matter in subsequent processes.

In particular, the marking part 8 performs marking in a state where the sample S is stopped or moving at a speed slower than the unwinding speed by the intermittent transfer part 4. Therefore, the portion where the foreign matter is detected can be marked directly and accurately in a stopped state or slowly moving state.

Additionally, the marking part 8 performs marking only on the portion where the foreign matter meeting predetermined conditions is detected or the vicinity thereof based on results of analysis of the foreign matter analyzed by the X-ray analysis part 5, or performs marking with a specific mark. Therefore, for example, a portion where a foreign matter that do not cause a defective product is detected and a portion where a foreign matter that causes a defective product is detected can be distinguished by the presence of marking or the difference in marks.

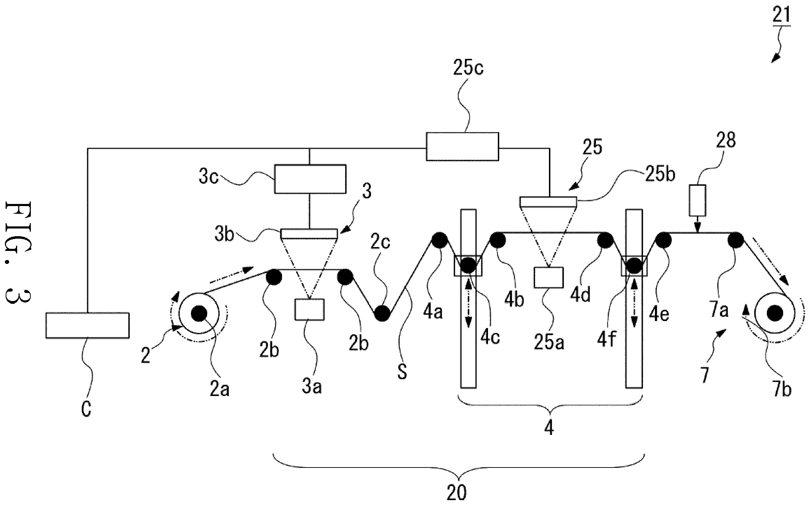

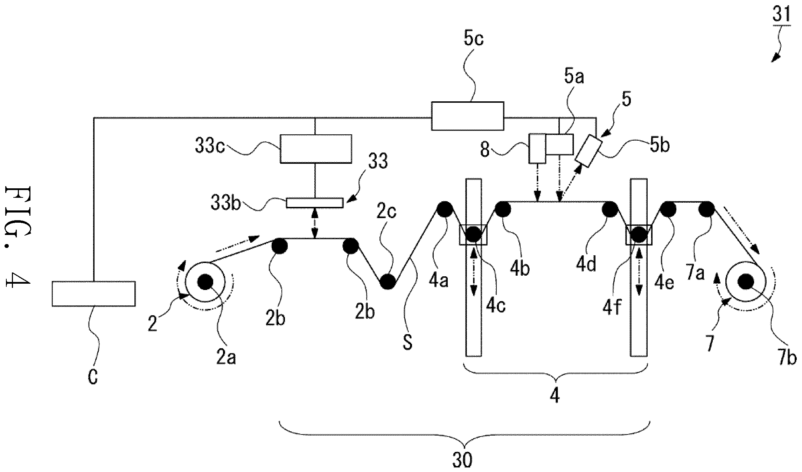

Next, second and third embodiments of an X-ray analysis apparatus according to the present disclosure will be described below with reference to FIGS. 3 and 4. In describing each embodiment below, the same components described in the above embodiment are given the same reference numerals, and descriptions thereof are omitted.

The second embodiment and the first embodiment are different in the following aspect. In the first embodiment, the X-ray analysis part 5 is a fluorescent X-ray analysis part, whereas in an X-ray analysis apparatus 21 according to the second embodiment, as illustrated in FIG. 3, both a foreign matter location detection part 3 and an X-ray analysis part 25 are transmission X-ray inspection parts.

That is, in the second embodiment, the foreign matter location detection part 3 of a foreign matter inspection and analysis part 20 is a first transmission X-ray inspection part that detects a foreign matter by irradiating a sample S with transmission X-rays, and the X-ray analysis part 25 is a second transmission X-ray inspection part that detects the size of the foreign matter by irradiating a portion where the foreign matter is detected with transmission X-rays.

The X-ray analysis part 25 according to the second embodiment includes a third X-ray source 25a disposed below the sample S between a second fixed roller 4b and a third fixed roller 4d of an intermittent transfer part 4; a third X-ray analyzer 25b disposed above the sample S between the second fixed roller 4b and the third fixed roller 4d so as to face the third X-ray source 25a; and a foreign matter analysis part 25c electrically connected to the third X-ray analyzer 25b and transmitting information on the size of the foreign matter.

Additionally, the third X-ray source 25a and the third X-ray analyzer 25b are movable to a location facing the location of the foreign matter by an analysis part moving mechanism such as an XY stage.

Additionally, in the second embodiment, a marking part 28 marks the portion where the foreign matter is detected only when the detected foreign matter is of size that causes a defective product.

The marking part 28 according to the second embodiment is disposed to enable marking on the sample S transferred between a fourth fixed roller 4e and a second guide roller 7a.

For example, in a case where the size (outer diameter) of the foreign matter is equal to or greater than 100 µm and causes a defective product, when the size of the foreign matter measured by the X-ray analysis part 25, which is the second transmission X-ray inspection part, is equal to or greater than 100 µm, the marking part 28 marks the location of the foreign matter or the vicinity location thereof, or marks it with a mark indicating NG. On the contrary, when the size of the foreign matter measured by the X-ray analysis part 25 is less than 100 µm, the marking part 28 does not perform marking or marks the location of the foreign matter or the vicinity location thereof with a mark indicating OK.

As described above, the X-ray analysis apparatus 21 according to the second embodiment has the following characteristics. The foreign matter location detection part 3 is the first transmission X-ray inspection part that detects the foreign matter by irradiating the sample S with transmission X-rays, and the X-ray analysis part 25 is the second transmission X-ray inspection part that detects the size of the foreign matter by irradiating the portion where the foreign matter is detected with transmission X-rays. Therefore, while the sample S is moving, the first transmission X-ray inspection part (foreign matter location detection part 3) performs rough detection of only the location of the foreign matter, and then in a state while the sample S is stopped or moving at a speed slower than the unwinding speed, the second transmission X-ray inspection part (X-ray analysis part 25) detects the size of the foreign matter. In this manner, a more detailed analysis of the foreign matter can be performed in two steps.

Next, the third embodiment and the first embodiment are different in the following aspect. In the first embodiment, the foreign matter location detection part 3 is the transmission X-ray inspection part, whereas in an X-ray analysis apparatus 31 according to the third embodiment, as illustrated in FIG. 4, a foreign matter location detection part 33 of a foreign matter inspection and analysis part 30 is a visible light inspection part that detects a foreign matter by detecting visible light from a sample S.

That is, the foreign matter location detection part 3 according to the third embodiment is a visible light inspection part disposed above the sample S transferred between a pair of foreign matter inspection rollers 2b, and includes a visible light measurement device 33b, such as a CCD camera, capturing an image of a surface of the sample S and a foreign matter determination part 33c detecting a foreign matter from the image of the sample S.

The foreign matter location detection part 33 captures an image of the state of the surface of the sample S with the visible light measurement device 33b, detects a foreign matter such as dust, wrinkles, bubbles, or discoloration through the foreign matter determination part 33c, and transmits the location of the foreign matter as foreign matter information to a controller C.

As described above, the X-ray analysis apparatus 31 according to the third embodiment has the following characteristics. The foreign matter location detection part 33 is the visible light inspection part that detects the foreign matter by detecting visible light from the sample S. Therefore, the visible light inspection part (foreign matter location detection part 33) can check the foreign matter, such as dust, wrinkles, bubbles, or discoloration, of the sample S with visible light, and the fluorescent X-ray analysis part (X-ray analysis part 5) can identify elements constituting the foreign matter.

Additionally, the present disclosure should not be construed as being limited to only the above-described embodiments and may be embodied in many different forms without departing from the spirit and scope of the present disclosure.

For example, in the foreign matter analysis part according to the first embodiment, an X-ray source and an X-ray analyzer for fluorescent X-ray analysis are disposed above the sample. However, the present disclosure is not limited thereto, the X-ray source and the X-ray analyzer for fluorescent X-ray analysis may be disposed below the sample. In this case, even when the sample is thick, it is possible to perform elemental identification with high accuracy not only for a foreign matter on the surface side of the sample but also for a foreign matter on the back surface side of the sample.

### [Explanation of reference number]

- 1, 21, 31: X-ray analysis apparatus
- 2: unwinding part
- 3, 33: foreign matter location detection part
- 4: intermittent transfer part
- 5, 25: X-ray analysis part
- 7: winding part
- 8, 28: marking part
- 10, 20, 30: foreign matter inspection and analysis part
- S: sample

## Claims

1. An X-ray analysis apparatus, comprising:
an unwinding part (2) continuously unwinding a sample (S), which is a sheet in strip-shape, downstream at a constant unwinding speed;
a foreign matter inspection and analysis part (10, 20, 30) inspecting and analyzing a foreign matter in the sample (S) unwound from the unwinding part (2); and
a winding part (7) winding the sample (S) that passed through the foreign matter inspection and analysis part (10, 20, 30),
**characterized in that** the foreign matter inspection and analysis part (10, 20, 30) comprises: a foreign matter location detection part (3) detecting a location of the foreign matter in the sample (S) that is unwound from the unwinding part (2) and moving;
an intermittent transfer part (4) intermittently stopping or slowing movement of a portion of the sample (S) where the foreign matter is detected to a speed slower than the unwinding speed downstream of the foreign matter location detection part (3) when the foreign matter is detected by the foreign matter location detection part (3); and
an X-ray analysis part (5) irradiating, with X-rays, the portion where the foreign matter is detected that is stopped or moving at the speed slower than the unwinding speed and performing analysis,
wherein the intermittent transfer part (4) discharges the portion where the foreign matter is detected that is stopped or moving at the speed slower than the unwinding speed to the winding part (7) after the analysis.

2. The X-ray analysis apparatus according to claim 1, wherein the foreign matter location detection part (3) is a transmission X-ray inspection part that detects the foreign matter by irradiating the sample (S) with transmission X-rays, and
the X-ray analysis part (5) is a fluorescent X-ray analysis part that detects fluorescent X-rays emitted from the sample (S) by irradiating the portion where the foreign matter is detected with X-rays.

3. The X-ray analysis apparatus according to claim 1, wherein the foreign matter location detection part (3) is a first transmission X-ray inspection part that detects the foreign matter by irradiating the sample (S) with transmission X-rays and
the X-ray analysis part (5) is a second transmission X-ray inspection part (25) that detects size of the foreign matter by irradiating the portion where the foreign matter is detected with X-rays.

4. The X-ray analysis apparatus according to claim 1, wherein the foreign matter location detection part (3) is a visible light inspection part (33) that detects the foreign matter by detecting visible light from the sample (S), and
the X-ray analysis part (5) is a fluorescent X-ray analysis part that detects fluorescent X-rays emitted from the sample (S) by irradiating the portion where the foreign matter is detected with X-rays.

5. The X-ray analysis apparatus according to any one of claims 1 to 4, further comprising:
a marking part (8, 28) marking the portion where the foreign matter is detected or a vicinity thereof.

6. The X-ray analysis apparatus according to claim 5, wherein the marking part (8, 28) performs marking in a state where the sample (S) is stopped or moving at a speed slower than the unwinding speed by the intermittent transfer part (4).

7. The X-ray analysis apparatus according to claim 5, wherein the marking part (8, 28) performs marking only on the portion where the foreign matter meeting predetermined conditions is detected or a vicinity thereof based on results of analysis of the foreign matter analyzed by the X-ray analysis part (5) or performs marking with a specific mark.
